# EUROPEAN PATENT APPLICATION

(11) **EP 3 967 317 A1**
(43) Date of publication of application: **16.03.2022**
(21) Application number: 19927624.7
(22) Date of filing: 13.05.2019
(51) Int. Cl.: A61K 35/745

(54) **USE OF INTESTINAL BACTERIA IN PREPARATION OF DRUG FOR PROMOTING TCR ??+T CELL PROLIFERATION**

(30) Priority: 07.05.2019 CN 201910375519
(71) Applicant: Revaissant (Shenzhen) Biosciences Co., Ltd., Guangdong 518172 (CN)
(72) Inventor: CAI, Qingqing, Shenzhen, Guangdong 518054 (CN)
(74) Representative: HGF
(86) International application number: PCT/CN2019/086598
(87) International publication number: WO 2020/223988

(57) **Abstract**

Provided is a use of any one or more of intestinal bacteria selected from a group consisting of *Alistipes shahii, Eubacterium ramulus, Eubacterium siraeum, Prevotella ruminicola, Ruminococcu lactaris, Anaerofustis stercorihominis, Clostridium nexile*, *Sanguibacteroides justesenii, Dorea formicigenerans*, and *Bifidobacterium saeculare* in preparation of a medicament for promoting an accumulation of CD8 positive cytotoxic T lymphocytes and/or a proliferation of TCR γδ+ T cells and/or preventing and/or treating tumors. Also provided is a pharmaceutical composition, foods, health products and food additives comprising one or more of the above-mentioned intestinal bacteria for promoting an accumulation of CD8 positive cytotoxic T lymphocytes and/or a proliferation of TCR γδ⁺ T cells and/or preventing and/or treating tumors.

## Description

### FIELD OF TECHNOLOGY

The present disclosure relates to the technical field of immunology, particularly, it relates to at least one of a use of intestinal bacteria in preparation of a medicament for promoting a proliferation of TCR yδ⁺ T cells, a use of intestinal bacteria in preparation of a medicament for promoting an accumulation of CD8 positive cytotoxic T lymphocytes, or a use of intestinal bacteria in preparation of a medicament for preventing and/or treating cancers, and at least one of a composition for promoting a proliferation of TCR yδ⁺ T cells, a composition for promoting an accumulation of CD8 positive cytotoxic T lymphocytes, or a composition for preventing and/or treating cancers.

### BACKGROUND

Cancer has become a serious problem threating human health and livelihood. The latest global statistics from the World Health Organization (WHO) show that cancer is among the leading cause of death worldwide. In 2018, there were 1.81 million new cases and about 0.96 million cancer-related deaths around the world. The morbidity and mortality of cancer have increased rapidly. And in 2018, liver cancer had been the fifth leading cause of death globally, and there were 0.627 million liver cancer deaths, accounting for 8.2% of all cancer deaths. Traditional treatment methods comprise surgical treatment, chemotherapy, radiotherapy, traditional Chinese medicine (TCM) therapy and the like, which are widely used in clinical and have reliable curative effects. However, these treatment methods have impacts on the quality of life for patients to different degrees. Particularly, most tumors, like liver cancer, are found at an advanced stage and are not suitable for the radical surgical-treatment. Therefore, it is urgent to develop safer and more effective treatment methods.

Since tumor immunotherapy is listed as the most significant breakthrough in annual scientific research by Science in 2013, a biological immunotherapy for tumors has developed rapidly. The tumor immunotherapy mainly refers to achieve anti-tumor effects by increasing the activity of immune system in body and relieving immunosuppression. However, most of patients have poor efficacy, because CD8 positive cytotoxic T lymphocytes in tumor microenvironment are so insufficient that they are unable to provide effective immune response. Therefore, it is urgent to find an immunotherapy approach to effectively modulate the immune system. T cells can be classified into αβ T cells and γδ T cells according to receptors on the surface of the T cells. The receptors on the surface of γδ T cells consist of two glycoprotein chains, γ chain and δ chain. The γδ T cells can directly identify proteins, peptides, or non-peptide antigens without antigen presentation by histocompatibility complex molecule on the surface of cell, which is an important biological feature. The γδ T cells are mainly located on skins and mucosal tissues, such as alimentary tract, respiratory tract, reproductive system, etc. At present, it is found in some studies that the γδ T cells can indirectly promote anti-tumor immune response for many malignant cancers by cytotoxicity or by stimulating and regulating the biological functions of other immune cells, for example dendritic cells, or CD8 positive T cells. However, it has not been found whether ten types of intestinal bacteria, such as *Alistipes shahii* and the like can promote an effective accumulation and an effective proliferation of CD8 positive cytotoxic T lymphocytes and/or γδ T cells (TCR γδ⁺ T cells).

The human intestinal bacteria have significant regulatory effects on the body and play an important role in the occurrence and development of the diseases. There are millions of bacteria living in human gut where anaerobic bacteria dominate. The present research indicates that intestinal bacteria play a powerful role in exhibiting anti-tumor effects by stimulating the activity of immune system in body and eliminating immunosuppression for tumor cells. *Alistipes shahii* is a Gram-negative, obligate anaerobe with rod shape. *Eubacteria* is a Gram-negative, motile, non-spore-forming, obligate anaerobe, which ferments glucose or peptone to produce substances mainly including a large amount of butyric acid, acetic acid, or formic acid. *Eubacteria* is found in animal cavity, faeces, animal and plant products, and soils. *Ruminococcus* is one type of *Clostridium. Anaerofustis* is a Gran-positive, non-spore obligate anaerobe with rod shape. *Prevotella copri* is a Gram-negative anaerobe and a symbiotic human gut bacterium, which is found to be relevant to the susceptibility of rheumatoid arthritis and the insulin resistance of the diabetic.

### SUMMARY

An objective of the present disclosure is to provide a technical solution for effectively promoting a proliferation of TCR γδ⁺ T cells and an accumulation of CD8 positive cytotoxic T lymphocytes in prevention and/or treatment of tumors.

For this purpose, the present disclosure provides a use of any one or more of intestinal bacteria selected from a group consisting of *Alistipes shahii, Eubacterium ramulus, Eubacterium siraeum, Prevotella ruminicola, Ruminococcu lactaris, Anaerofustis stercorihominis, Clostridium nexile, Sanguibacteroides justesenii, Dorea formicigenerans,* and *Bifidobacterium saeculare* in preparation of a medicament for promoting a proliferation of TCR γδ⁺ T cells (γδ cells for short).

Preferably, the intestinal bacteria are any one or more of live intestinal bacteria; genetically recombined, altered or modified, attenuated, chemically treated, physically treated, or inactivated intestinal bacteria; lysate of intestinal bacteria; and/or culture supernatant of intestinal bacteria.

Preferably, an increase in the proliferation of TCR γδ⁺ T cells is characterized by an increase in a relative amount of TCR γδ⁺ T cells in cecum cells.

Accordingly, the present disclosure also provides a composition for promoting a proliferation of TCR γδ⁺ T cells, comprising any one or more of intestinal bacteria selected from a group consisting of *Alistipes shahii, Eubacterium ramulus, Eubacterium siraeum, Prevotella ruminicola, Ruminococcu lactaris, Anaerofustis stercorihominis, Clostridium nexile, Sanguibacteroides justesenii, Dorea formicigenerans,* and *Bifidobacterium saeculare* as an active ingredient.

Preferably, the intestinal bacteria are any one or more of live intestinal bacteria; genetically recombined, altered or modified, attenuated, chemically treated, physically treated, or inactivated intestinal bacteria; lysate of intestinal bacteria; and/or culture supernatant of intestinal bacteria.

Another aspect of the present disclosure is to provide a use of any one or more of intestinal bacteria selected from a group consisting of *Alistipes shahii, Eubacterium ramulus, Eubacterium siraeum, Prevotella ruminicola, Ruminococcu lactaris*, *Anaerofustis stercorihominis, Clostridium nexile, Sanguibacteroides justesenii, Dorea formicigenerans*, and *Bifidobacterium saeculare* in preparation of a medicament for accumulating CD8 positive cytotoxic T lymphocytes.

Preferably, the intestinal bacteria are any one or more of live intestinal bacteria; genetically recombined, altered or modified, attenuated, chemically treated, physically treated, or inactivated intestinal bacteria; lysate of intestinal bacteria; and/or culture supernatant of intestinal bacteria.

Preferably, the accumulation of CD8 positive cytotoxic T lymphocytes is characterized by an increase in a relative amount of CD8 positive cytotoxic T lymphocytes in tumor microenvironment

Accordingly, the present disclosure also provides a composition for promoting an accumulation of CD8 positive cytotoxic T lymphocytes, comprising any one or more of intestinal bacteria selected from a group consisting of *Alistipes shahii, Eubacterium ramulus, Eubacterium siraeum, Prevotella ruminicola, Ruminococcu lactaris*, *Anaerofustis stercorihominis, Clostridium nexile, Sanguibacteroides justesenii, Dorea formicigenerans*, and *Bifidobacterium saeculare* as an active ingredient.

Preferably, the intestinal bacteria are any one or more of live intestinal bacteria; genetically recombined, altered or modified, attenuated, chemically treated, physically treated, or inactivated intestinal bacteria; lysate of intestinal bacteria; and/or culture supernatant of intestinal bacteria.

The inventors of the present disclosure found that it is possible for ten types of intestinal bacteria such as *Alistipes shahii* to promote an accumulation of CD8 positive cytotoxic T lymphocytes and/or a proliferation of TCR γδ⁺ T cells in tumor so as to modulate T cell immune response. Specifically, the inventors found that, when any one or more of ten types of intestinal bacteria, or physiological active ingredients or compositions obtained from these bacteria are comprised in tumors, they can promote the proliferation of TCR γδ⁺ T cells and/or the accumulation of CD8 positive cytotoxic T lymphocytes in tumor microenvironment in cecum. Thus, anti-tumor effects of T cells are improved to achieve objectives of the present disclosure. Specifically, they are achieved by strengthening the functions of anti-tumor immune cell subsets.

Specifically, the CD8 positive cytotoxic T lymphocytes refer to CD8 T cells characterized by the phenotype of CD8⁺, and TCR γδ⁺ T cells (γδ TCR⁺ for short) refer to γδ T cells characterized by the phenotype of TCR γδ⁺.

Further, another aspect of the present disclosure is to provide a use of any one or more of intestinal bacteria selected from a group consisting of *Alistipes shahii, Eubacterium ramulus, Eubacterium siraeum, Prevotella ruminicola, Ruminococcu lactaris, Anaerofustis stercorihominis, Clostridium nexile, Sanguibacteroides justesenii, Dorea formicigenerans*, and *Bifidobacterium saeculare* in preparation of a medicament for preventing and/or treating tumors.

Preferably, the intestinal bacteria are any one or more of live intestinal bacteria; genetically recombined, altered or modified, attenuated, chemically treated, physically treated, or inactivated intestinal bacteria; lysate of intestinal bacteria; and/or culture supernatant of intestinal bacteria.

Preferably, the tumor is a solid tumor.

Accordingly, the present disclosure also provides a composition for preventing and/or treating tumors, comprising any one or more of intestinal bacteria selected from a group consisting of *Alistipes shahii, Eubacterium ramulus, Eubacterium siraeum, Prevotella ruminicola, Ruminococcu lactaris, Anaerofustis stercorihominis, Clostridium nexile, Sanguibacteroides justesenii, Dorea formicigenerans*, and *Bifidobacterium saeculare* as an active ingredient.

Preferably, the intestinal bacteria are any one or more of live intestinal bacteria; genetically recombined, altered or modified, attenuated, chemically treated, physically treated, or inactivated intestinal bacteria; lysate of intestinal bacteria; and/or culture supernatant of intestinal bacteria.

More preferably, the composition includes but is not limited to any one of a pharmaceutical composition, a food, a health product, or a food additive.

It is another aspect of the present disclosure to provide a method of promoting a proliferation of TCR γδ⁺ T cells and/or promoting an accumulation of CD8 positive cytotoxic T lymphocytes, and/or preventing and or treating tumors.

Specifically, the present disclosure provides a method of stimulating a proliferation of TCR γδ⁺ T cells, and/or an accumulation of CD8 positive cytotoxic T lymphocytes, and/or preventing and/or treating tumors in a subject (for example, the subject in need thereof, for example, those in need of stimulating the accumulation of CD8 positive cytotoxic T lymphocytes, and/or the proliferation of TCR γδ⁺ T cells, and/or preventing and/or treating tumors). The method comprises the step of administrating the composition to a subject, wherein the composition comprises any one or more of intestinal bacteria selected from a group consisting of *Alistipes shahii, Eubacterium ramulus, Eubacterium siraeum, Prevotella ruminicola, Ruminococcu lactaris*, *Anaerofustis stercorihominis, Clostridium nexile, Sanguibacteroides justesenii, Dorea formicigenerans*, and *Bifidobacterium saeculare* as an active ingredient.

More specifically, the method comprises the steps as follows:
(1) administrating a composition to a subject, wherein the composition comprises any one or more intestinal bacteria selected from a group consisting of *Alistipes shahii, Eubacterium ramulus, Eubacterium siraeum, Prevotella ruminicola, Ruminococcu lactaris*, *Anaerofustis stercorihominis, Clostridium nexile*, *Sanguibacteroides justesenii, Dorea formicigenerans*, and *Bifidobacterium saeculare* as an active ingredient;
(2) stimulating an accumulation of CD8 positive cytotoxic T lymphocytes and/or a proliferation of TCR γδ⁺ T cells as follows:
   administrating the composition every three days for stimulation, and then transplanting tumors ten days later, followed by further administrating the composition to measure the amount of CD8 positive cytotoxic T lymphocytes and TCR γδ⁺ T cells in the subject, wherein Day zero refers to the very first day for administrating the composition; and
(3) measuring the amount of CD8 positive cytotoxic T lymphocytes and TCR γδ⁺ T cells as follows:
   detecting the proportion of CD8 T cells and TCR γδ +T cells in culture cells by flow cytometry, with TCR γδ and CD8 as molecular markers, wherein the CD8 positive T cell is characterized by the phenotype of CD8⁺ and TCR γδ⁺ T cells is characterized by the phenotype of TCR γδ⁺.

According to the method, the measurement result of CD8⁺ is used as an indicator for the proliferation or accumulation of CD8 positive cytotoxic T lymphocytes, and the measurement result of TCR γδ⁺ T is used as an indicator for the proliferation or accumulation of TCR γδ⁺ T cells.

The composition of the present disclosure comprises any one or more of ten types of intestinal bacteria as an active ingredient and it is an excellent composition for stimulating the accumulation or proliferation of CD8 positive cytotoxic T lymphocytes and/or TCR γδ⁺ T cells. Immunity in body can be increased through administering a pharmaceutical product that comprises the composition of the present disclosure, or taking foods or drinks that comprises the composition of the present disclosure. Therefore, the composition of the present disclosure can be used for such as preventing or treating infectious disease, autoimmune disease, or allergic disease, etc. In addition, if the composition of the present disclosure is comprised in the foods or drinks such as health food, healthy individuals can take the composition easily and routinely. Thus, it is possible to stimulate the accumulation or proliferation of CD8 positive cytotoxic T lymphocytes and/or TCR γδ⁺ T cells to improve the immunity.

### BRIEF DESCRIPTION

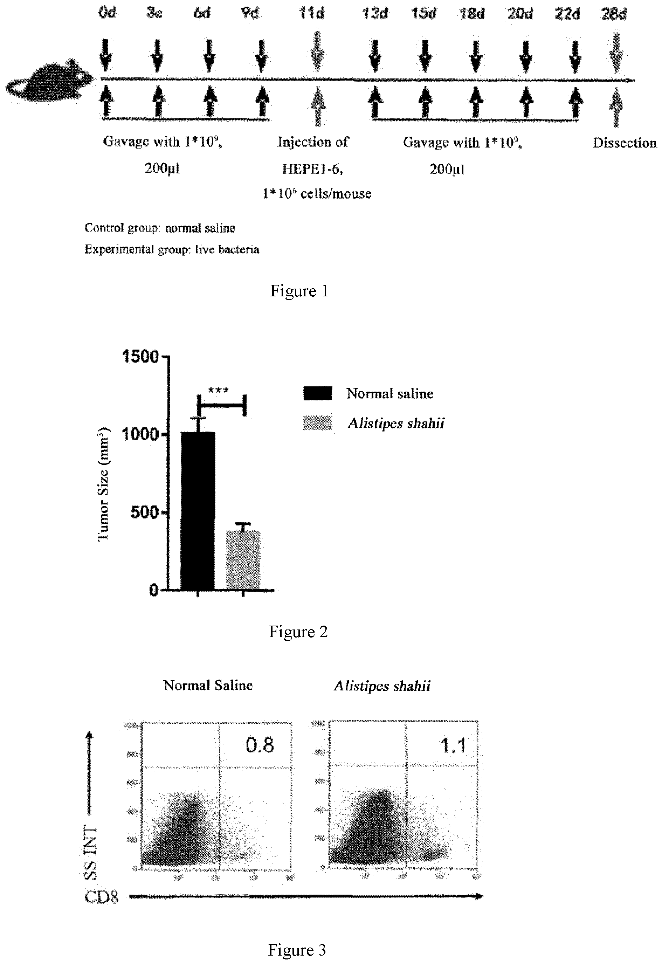

Figure 1 depicts a schematic flow diagram of an experiment of ten types of intestinal bacteria for promoting an accumulation of CD8 positive cytotoxic T lymphocytes and/or TCR γδ⁺ T cells, and for treating tumors in a mouse model of liver cancer. Time is expressed in days (Day, d) for administrating the intestinal bacteria or transplanting tumor cells.
Figure 2 depicts a statistical analysis graph of tumor sizes in three typical mice transplanted with liver cancer cells in each group, after intestinal bacteria (for example *Alistipes shahii,* but intestinal bacteria being not limited to *Alistipes shahii)* are administrated.
Figure 3 depicts an analysis graph of flow cytometry of CD8 positive cytotoxic T lymphocytes in one typical mouse in each group, after intestinal bacteria (for example *Alistipes shahii,* but intestinal bacteria being not limited to *Alistipes shahii)* are administrated to mice transplanted with liver cancer cells, wherein right lower quadrant indicates CD8 positive cytotoxic T lymphocytes, and numerics in the right lower quadrant show the percentage of CD8 positive cytotoxic T lymphocytes in total cells in cecum.

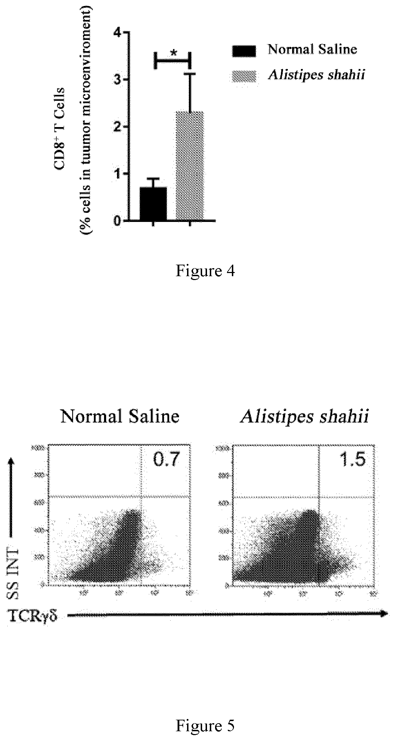

Figure 4 depicts a statistical analysis graph of the percentage of CD8 positive cytotoxic T lymphocytes in total cells in cecum, after intestinal bacteria (for example *Alistipes shahii,* but intestinal bacteria being not limited to *Alistipes shahii)* are administrated to mice transplanted with liver cancer cells.
Figure 5 depicts an analysis graph of flow cytometry of TCR γδ positive T lymphocytes in one typical mouse in each group, after intestinal bacteria (for example *Alistipes shahii,* but intestinal bacteria being not limited to *Alistipes shahii)* are administrated to mice transplanted with liver cancer cells, wherein right lower quadrant indicates TCR γδ positive T lymphocytes, and numerics in the right lower quadrant show the percentage of TCR γδ positive T lymphocytes in total cells in cecum.

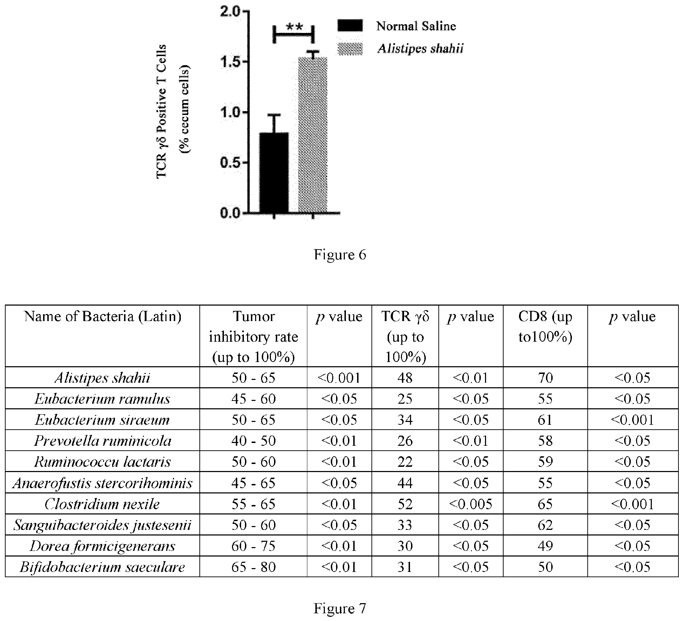

Figure 6 depicts a statistical analysis graph of the percentage of TCR γδ positive T lymphocytes in total cells in cecum after intestinal bacteria (for example *Alistipes shahii,* but intestinal bacteria being not limited to *Alistipes shahii)* are administrated to mice transplanted with liver cancer cells.
Figure 7 depicts a statistical analysis table showing tumor inhibitory rate, an increased percentage of CD8 positive cytotoxic T lymphocytes in tumor and an increased percentage of TCR γδ positive T lymphocytes in cecum, after ten types of intestinal bacteria are administrated to mice transplanted with liver cancer cells.

### DETAILED DESCRIPTION

The present disclosure will be further described below with reference to the specific embodiments. It should be pointed out that ten types of intestinal bacteria (such as *Alistipes shahii),* or pharmaceutical compositions, foods, health products and food additives of the present disclosure comprising ten types of intestinal bacteria (such as *Alistipes shahii)* for promoting an accumulation of CD8 positive cytotoxic T lymphocytes and/or a proliferation of TCR γδ⁺ T lymphocytes, can be applied to indications described above and exhibits the functions described above, after being administrated to a test subject. All dosage forms within the scope of the present disclosure have been tested, and only small parts of them are described hereinafter in the examples for illustration, however, they should not be understood as a limitation of the invention.

The present disclosure provides a composition comprising any one or more of ten types of intestinal bacteria for promoting an accumulation of CD8 positive cytotoxic T lymphocytes and/or a proliferation of TCR γδ positive T lymphocytes, wherein the composition comprises at least one of ten types of live intestinal bacteria; ten types of genetically recombined, altered or modified, attenuated, chemically treated, physically treated, or inactivated intestinal bacteria; ten types of lysates of intestinal bacteria; and/or ten types of culture supernatants of intestinal bacteria as an active ingredient.

There is no specific limitation to "any one or more of ten types of intestinal bacteria, such as *Alistipes shahii,* etc" acting as an active ingredient of the composition described in the present disclosure, as long as the bacteria can be used for promoting an accumulation of CD8 positive cytotoxic T lymphocytes and/or a proliferation of TCR γδ positive T lymphocytes. The ten types of intestinal bacteria, such as *Alistipes shahii,* etc, can be used individually in the composition of the present disclosure and can be combined with other bacteria excluding these bacteria as well.

In the disclosure, the "CD8 positive T cells" refers to T lymphocytes expressing CD8 to participate in immune response. The "γδ T cells" refers to T lymphocytes expressing TCR γ chain and/or TCR δ chain to participate in immune response.

The present disclosure will be further described with embodiments as follows, but is not limited to the scope of the embodiments described herein.

### Example 1: Culture of Intestinal bacteria

### 1. Culture method of Alistipes shahii

Step 1: A cryopreserved *Alistipes shahii* strain (purchased from ATCC official website) was taken and then 200 uL of Trypticase Soy Agar (TSA) culture medium was added to redissolve it to obtain a bacterial solution. Subsequently, 20 uL of the bacterial solution was pipetted and streaked on a blood agar plate. After an air exhaustion processing by an anaerobic jar gassing system, the agar plate was placed in an incubator and incubated anaerobically at 37 ° C for 48 h;
Step 2: A monoclonal colony was selected to inoculate in 10 mL of TSA culture medium, and incubated anaerobically at 37 ° C for 12 h;
Step 3: 1% (v/v) of strains were inoculated in 500 ml of TSA culture medium in a flask, incubated anaerobically at 37 ° C for 48 h;
Step 4: The bacterial solution was collected, and centrifuged at 6000 rpm for 10 min, washed twice with saline. Finally, the bacterial sludge was redissolved with saline for later use and viable bacteria were counted.

### 2. Culture method of Eubacterium ramulus or Eubacterium siraeum

Step 1: A cryopreserved *Eubacterium (Eubacterium ramulus* or *Eubacterium siraeum)* strain (purchased from ATCC official website) was taken and 200 uL of TSA culture medium was added to redissolve it to obtain a bacterial solution. Subsequently, 20 uL of the bacterial solution was pipetted and streaked on a blood agar plate. After an air exhaustion processing by an anaerobic jar gassing system, the agar plate was placed in an incubator and incubated anaerobically at 37 ° C for 48 h;
Step 2: A monoclonal colony was selected to inoculate in 10 mL of TSA culture medium, incubated anaerobically at 37 ° C for 12 h;
Step 3: 1% (v/v) of strains were inoculated in 500 ml of TSA culture medium in a flask, and incubated anaerobically at 37 ° C for 48 h;
Step 4: The bacterial solution was collected, and centrifuged at 6000 rpm for 10 min, washed twice with saline. Finally, the bacterial sludge was redissolved with saline for later use and viable bacteria were counted.

### 3. Culture method of Prevotella ruminicola

Step 1: A cryopreserved *Prevotella ruminicola* strain (purchased from ATCC official website) was taken and 200 uL of TSA culture medium was added to redissolve it to obtain a bacterial solution. Subsequently, 20 uL of the bacterial solution was pipetted and streaked on a blood agar plate. After an air exhaustion processing by an anaerobic jar gassing system, the agar plate was placed in an incubator and incubated anaerobically at 37 ° C for 48 h;
Step 2: A monoclonal colony was selected to inoculate in 10 mL of TSA culture medium, incubated anaerobically at 37 ° C for 12 h;
Step 3: 1% (v/v) of strains were inoculated in 500 ml of TSA culture medium in a flask, and incubated anaerobically at 37 ° C for 48 h;
Step 4: The bacterial solution was collected, and centrifuged at 6000 rpm for 10 min, washed twice with saline. Finally, the bacterial sludge was redissolved with saline for later use and viable bacteria were counted.

### 4. Culture method of Ruminococcu lactaris

Step 1: A cryopreserved *Ruminococcu lactaris* strain (purchased from ATCC official website) was taken and 200 uL of TSA culture medium was added to redissolve it to obtain a bacterial solution. Subsequently, 20 uL of the bacterial solution was pipetted and streaked on a blood agar plate. After an air exhaustion processing by an anaerobic jar gassing system, the agar plate was placed in an incubator and incubated anaerobically at 37 ° C for 48 h;
Step 2: A monoclonal colony was selected to inoculate in 10 mL of TSA culture medium, incubated anaerobically at 37 ° C for 12 h;
Step 3: 1% (v/v) of strains were inoculated in 500 ml of TSA culture medium in a flask, and incubated anaerobically at 37 ° C for 48 h;
Step 4: The bacterial solution was collected, and centrifuged at 6000 rpm for 10 min, washed twice with saline. Finally, the bacterial sludge was redissolved with saline for later use and viable bacteria were counted.

### 5. Culture method of Anaerofustis stercorihominis

Step 1: A cryopreserved *Anaerofustis stercorihominis* strain (purchased from ATCC official website) was taken and 200 uL of TSA culture medium was added to redissolve it to obtain a bacterial solution. Subsequently, 20 uL of the bacterial solution was pipetted and streaked on a blood agar plate. After an air exhaustion processing by an anaerobic jar gassing system, the agar plate was placed in an incubator and incubated anaerobically at 37 ° C for 48 h;
Step 2: A monoclonal colony was selected to inoculate in 10 mL of TSA culture medium, incubated anaerobically at 37 ° C for 12 h;
Step 3: 1% (v/v) of strains were inoculated in 500 ml of TSA culture medium in a flask, and incubated anaerobically at 37 ° C for 48 h;
Step 4: The bacterial solution was collected, and centrifuged at 6000 rpm for 10 min, washed twice with saline. Finally, the bacterial sludge was redissolved with saline for later use and viable bacteria were counted.

### 6. Culture method of Clostridium nexile

Step 1: A cryopreserved *Clostridium nexile* strain (purchased from ATCC official website) was taken and 200 uL of TSA culture medium was added to redissolve it to obtain a bacterial solution. Subsequently, 20 uL of the bacterial solution was pipetted and streaked on a blood agar plate. After an air exhaustion processing by an anaerobic jar gassing system, the agar plate was placed in an incubator and incubated anaerobically at 37 ° C for 48 h;
Step 2: A monoclonal colony was selected to inoculate in 10 mL of TSA culture medium, incubated anaerobically at 37 ° C for 12 h;
Step 3: 1% (v/v) of strains were inoculated in 500 ml of TSA culture medium in a flask, and incubated anaerobically at 37 ° C for 48 h;
Step 4: The bacterial solution was collected, and centrifuged at 6000 rpm for 10 min, washed twice with saline. Finally, the bacterial sludge was redissolved with saline for later use and viable bacteria were counted.

### 7. Culture method of Sanguibacteroides justesenii

Step 1: A cryopreserved *Sanguibacteroides justesenii* strain (purchased from ATCC official website) was taken and 200 uL of TSA culture medium was added to redissolve it to obtain a bacterial solution. Subsequently, 20 uL of the bacterial solution was pipetted and streaked on a blood agar plate. After an air exhaustion processing by an anaerobic jar gassing system, the agar plate was placed in an incubator and incubated anaerobically at 37° C for 48 h;
Step 2: A monoclonal colony was selected to inoculate in 10 mL of TSA culture medium, incubated anaerobically at 37° C for 12 h;
Step 3: 1% (v/v) of strains were inoculated in 500 ml of TSA culture medium in a flask, and incubated anaerobically at 37° C for 48 h;
Step 4: The bacterial solution was collected, and centrifuged at 6000 rpm for 10 min, washed twice with saline. Finally, the bacterial sludge was redissolved with saline for later use and viable bacteria were counted.

### 8. Culture method of Dorea formicigenerans

Step 1: A cryopreserved *Dorea formicigenerans* strain (purchased from ATCC official website) was taken and 200 uL of RGCA culture medium was added to redissolve it to obtain a bacterial solution. Subsequently, 20 uL of the bacterial solution was pipetted and streaked on a blood agar plate. After an air exhaustion processing by an anaerobic jar gassing system, the agar plate was placed in an incubator and incubated anaerobically at 37° C for 48 h;
Step 2: A monoclonal colony was selected to inoculate in 10 mL of RGCA culture medium, incubated anaerobically at 37° C for 12 h;
Step 3: 1% (v/v) of strains were inoculated in 500 ml of RGCA culture medium in a flask, and incubated anaerobically at 37 ° C for 48 h;
Step 4: The bacterial solution was collected, and centrifuged at 6000 rpm for 10 min, washed twice with saline. Finally, the bacterial sludge was redissolved with saline for later use and viable bacteria were counted.

### 9. Culture method of Bifidobacterium saeculare

Step 1: A cryopreserved *Bifidobacterium saeculare* strain (purchased from ATCC official website) was taken and 200 uL of Oxoid CM149 culture medium was added to redissolve it to obtain a bacterial solution. Subsequently, 20 uL of the bacterial solution was pipetted and streaked on a blood agar plate. After an air exhaustion processing by an anaerobic jar gassing system, the agar plate was placed in an incubator and incubated anaerobically at 37° C for 48 h;
Step 2: A monoclonal colony was selected to inoculate in 10 mL of Oxoid CM149 culture medium, incubated anaerobically at 37° C for 12 h;
Step 3: 1% (v/v) of strains were inoculated in 500 ml of Oxoid CM149 culture medium in a flask, and incubated anaerobically at 37° C for 48 h;
Step 4: The bacterial solution was collected, and centrifuged at 6000 rpm for 10 min, washed twice with saline. Finally, the bacterial sludge was redissolved with saline for later use and viable bacteria were counted.

### Example 2 An experiment of the above-mentioned intestinal bacteria (for example Alistipes shahii, but intestinal bacteria being not limited to Alistipes shahii) for stimulating an expression of γδ TCR and a proliferation of γδ T lymphocytes, and treating tumors in mice

### 1. Culture Method

A culture method of *Alistipes shahii* is the same as that in Example 1.

### 2. Sample Preparation

Preparation method of *Alistipes shahii* is the same as that in Example 1.

### 3. An experiment of an increased expression of γδ T lymphocytes in mice transplanted with liver tumors, stimulated by Alistipes shahii

Figure 1 is a schematic flow diagram of an experiment of detecting an increased proliferation of TCR γδ⁺ T lymphocytes in mice transplanted with liver tumor, stimulated by *Alistipes shahii.*

Experimental animal: Eighteen C57BL/6 mice aged 3-4 weeks in a good mental state were purchased from the Experimental Animal Center of Sun Yat-sen University. The mice were randomly separated into two groups, 9 mice in each group, and the two groups were a control group and a live bacteria gavage group, respectively. The two groups of mice were administered with live *Alistipes shahii* (10⁹ CFU) and normal saline (the control group) by gavage for two weeks, respectively. After Hep 1-6 mouse tumor (liver cancer) cells grew at logarithmic phase, the cells were digested with TE, and culture medium was neutralized. The cells were collected through centrifugation, and washed twice with DPBS to remove residual serum. After that, the cells were resuspended with DPBS. After the administration by gavage for about a week, the Hep 1-6 mouse tumor (liver cancer) cells were counted. Subsequently, each mouse was subcutaneously inoculated with 10⁶ cells into right axilla and continually treated by gavage, and then tumor-bearing mice were killed two weeks later. The size of tumors was statistically recorded, and the cells were collected, detected by using flow cytometry to analyze the amount of γδ T cells in cecum.

### Example 3 Detection of the amount of CD8 positive T cells by using flow cytometry

(1) The tumor cells were collected into a flow tube with 10⁷ cells in each tube, washed twice with PBS and centrifuged at 1500 rpm for 5 min, and then the cells were resuspended with 400 microliter of PBS after a removal of supernatant;
(2) An anti-CD8-FITC monoclonal antibody was added and mixed, and the tube was kept in a dark at 4 ° C for 30 min;
(3) The cells were washed twice with PBS, centrifuged at 1500 rpm for 5 min, and then the cells were suspended with 400 microliter of PBS after a removal of supernatant;
(4) Single cell suspension was detected on a flow cytometry machine, wherein CD8 positive T cells were characterized by the phenotype of CD8 positive.

### Example 4: Detection of the amount of yδ T cells by using flow cytometry

(1) The cecum cells were collected into a flow tube with 10⁷ cells in each tube, washed twice with PBS and centrifuged at 1500 rpm for 5 min, and then the cells were resuspended with 400 microliter of PBS after a removal of supernatant;
(2) An anti-TCRγδ-FITC monoclonal antibody was added and mixed, and the tube was kept in a dark at 4 ° C for 30 min;
(3) The cells were washed twice with PBS, centrifuged at 1500 rpm for 5 min, and then the cells were suspended with 400 microliter of PBS after a removal of supernatant;
(4) Single cell suspension was detected on a flow cytometry machine, wherein γδ T cells is characterized by the phenotype of TCR γδ positive.

### Analysis of Test Results

Figure 1 shows a schematic flow diagram of an experiment of intestinal bacteria for treating tumors in a mice transplanted with liver cancer. *Alistipes shahii* is taken as an example, but the intestinal bacteria are not limited to *Alistipes shahii.*

Figure 2 is a statistical analysis graph of tumor sizes in mice transplanted with liver cancer cells, after intestinal bacteria were administrated. *Alistipes shahii* is taken as an example, but the intestinal bacteria are not limited to *Alistipes shahii.*

It is clearly observed from the results of Figure 2 that, compared with the control group (normal saline), the tumor sizes in mice transplanted with liver cancer cells are significantly decreased by about 63% after *Alistipes shahii* was administrated, and the decrease represents statistically significant difference (*p*<0.001). Therefore, the administration of *Alistipes shahii* for treating the mice can significantly inhibit tumor growth. In other examples, it can also be observed that the tumor sizes are significantly decreased after the above-mentioned other nine types of intestinal bacteria were administrated, and the decrease represents statistically significant difference (p<0.005 to 0.05).

In other examples (experimental procedures and conditions are the same as above), tumor inhibitory rate of *Eubacterium ramulus* is about 45 to 60%, tumor inhibitory rate of *Eubacterium siraeum* is about 30 to 65%, tumor inhibitory rate of *Prevotella ruminicola* is about 40 to 50%, tumor inhibitory rate of *Ruminococcu lactaris* is about 50 to 60%, tumor inhibitory rate of *Anaerofustis stercorihominis* is about 45 to 65%, tumor inhibitory rate of *Clostridium nexile* is about 35 to 65%, tumor inhibitory rate of *Sanguibacteroides justesenii* is about 40 to 60%, tumor inhibitory rate of *Dorea formicigenerans* is about 50 to 75%, and tumor inhibitory rate of *Bifidobacterium saeculare* is about 35 to 80%,

Thus, all of the above-mentioned intestinal bacteria have a significant inhibitory effect on tumor growth.

Figure 3 is an analysis graph of flow cytometry of CD8 positive cytotoxic T lymphocytes for one typical mouse in each group in transplanted liver cancer cell microenvironment after the intestinal bacteria (for example *Alistipes shahii,* but intestinal bacteria being not limited to *Alistipes shahii)* are administrated to liver tumors in mice transplanted with liver cancer cells, wherein numerics in the right lower quadrant show the percentage of CD8 positive T lymphocytes in total cells in cecum. *Alistipes shahii* is taken as an example to obtain the statistical analysis graph of flow cytometry, but intestinal bacteria are not limited to *Alistipes shahii.*

It can be seen from the analysis graph of flow cytometry that, compared with the control group (normal saline), *Alistipes shahii* increases a relative amount of CD8⁺ T lymphocytes by about 70% in liver tumor microenvironment.

Figure 4 is a statistical analysis graph of the percentage of CD8 positive T lymphocytes in cells in tumor microenvironment of mice transplanted with liver cancer, three mice in each group.

It can be seen from the statistical analysis graph that, compared with the control group (pure water), *Alistipes shahii* increases a relative amount of CD8⁺ T lymphocytes in tumor microenvironment, and the increase represents statistically significant difference (*p*<0.001).

In other examples, the following nine types of intestinal bacteria, *Eubacterium ramulus, Eubacterium siraeum, Prevotella ruminicola, Ruminococcu lactaris, Anaerofustis stercorihominis, Clostridium nexile*, *Sanguibacteroides justesenii, Dorea formicigenerans*, and *Bifidobacterium saeculare* increase the percentage of CD8 positive T lymphocytes by about 55%, about 61%, about 58%, about 59%, about 55%, about 65%, about 62%, about 49%, and about 50%, respectively. Therefore, all the above-mentioned intestinal bacteria significantly increase a relative amount of CD8⁺ T lymphocytes in tumor microenvironment, and the increase represents statically significant difference (p<0.001 to 0.05).

Figure 5 is an analysis graph of flow cytometry of TCR γδ positive T lymphocytes in cecum in one typical mouse in each group, after the intestinal bacteria (for example *Alistipes shahii,* but intestinal bacteria being not limited to *Alistipes shahii)* are administrated to liver tumors in mice transplanted with liver cancer cells, wherein numerics in the right lower quadrant show the percentage of TCR γδ positive T lymphocytes in cecum cells.

It can be seen from the statistical analysis graph that, compared with the control group (normal saline), *Alistipes shahii* increases a relative amount of TCR γδ positive T lymphocytes by about 48% in liver tumor microenvironment.

Figure 6 is a statistical analysis graph of the percentage of TCR γδ positive T lymphocytes in cecum cells after the intestinal bacteria are administrated to mice transplanted with liver cancer cells, three mice in each group.

It can be seen from the statistical analysis graph that, compared with the control group (pure water), *Alistipes shahii* significantly increases a relative amount of TCR γδ⁺ T lymphocytes in cecum, and the increase represents statistically significant difference (*p*<0.001). *Alistipes shahii* is taken as an example to obtain the statistical analysis graph of flow cytometry, but intestinal bacteria are not limited to *Alistipes shahii.*

In other examples, the following nine types of intestinal bacteria, *Eubacterium ramulus, Eubacterium siraeum, Prevotella ruminicola, Ruminococcu lactaris*, *Anaerofustis stercorihominis, Clostridium nexile*, *Sanguibacteroides justesenii, Dorea formicigenerans*, and *Bifidobacterium saeculare* increase the percentage of TCR γδ positive T lymphocytes by about 25%, about 34%, about 26%, about 22%, about 44%, about 52%, about 33%, about 30%, and about 31%, respectively. Therefore, all the above-mentioned intestinal bacteria significantly increase a relative amount of TCR γδ⁺ T lymphocytes in cecum, and the increase represents statically significant difference (p<0.001 to 0.05).

In the statistical analysis graph, ^{∗} represents student *t*-test atp<0.05, ^{∗∗} represents student t-test at *p*<0.01. ^{∗∗∗} represents student t-test at *p*<0.0001. *p*<0.05 represents statistically significant difference.

In conclusion, ten types of intestinal bacteria used in the present disclosure have a significant inhibiting effect on tumor growth, and they significantly inhibit formation and growth of tumor in mice transplanted with liver cancer cells (Figure 2). It can be seen from the experiments of figures 3, 4, 5, 6 and 7 that, compared with the control group (normal saline), the live bacteria gavage group significantly increases the infiltration of CD8 positive cytotoxic T lymphocytes in tumor microenvironment and increases the accumulation of TCR γδ⁺ T lymphocytes in cecum, thereby significantly inhibiting tumor growth and having a good effect on the prevention and treatment of tumors.

The above content is a further detailed description of the technical solution in combination with the preferred embodiments of the present disclosure. It cannot be considered that the specific implementation of the present disclosure is limited to the above descriptions. For those skilled in the art, any simple deductions or replacements without departing from the inventive concept of the invention should all be regarded as belonging to the protection scope of the invention.

## Claims

1. Use of any one or more of intestinal bacteria selected from a group consisting of *Alistipes shahii, Eubacterium ramulus, Eubacterium siraeum, Prevotella ruminicola, Ruminococcu lactaris*, *Anaerofustis stercorihominis, Clostridium nexile, Sanguibacteroides justesenii, Dorea formicigenerans*, and *Bifidobacterium saeculare* in preparation of a medicament for promoting a proliferation of TCR γδ⁺ T cells.

2. The use according to claim 1, wherein the intestinal bacteria are any one or more of live intestinal bacteria; genetically recombined, altered or modified, attenuated, chemically treated, physically treated, or inactivated intestinal bacteria; lysate of intestinal bacteria; and/or culture supernatant of intestinal bacteria.

3. The use according to claim 1, wherein an increase in the proliferation of TCR γδ⁺ T cells is an increase in a relative amount of TCR γδ⁺ T cells in cecum cells.

4. A composition for promoting a proliferation of TCR γδ⁺ T cells, comprising any one or more of intestinal bacteria selected from a group consisting of *Alistipes shahii, Eubacterium ramulus, Eubacterium siraeum, Prevotella ruminicola, Ruminococcu lactaris*, *Anaerofustis stercorihominis, Clostridium nexile, Sanguibacteroides justesenii, Dorea formicigenerans*, and *Bifidobacterium saeculare* as an active ingredient.

5. The composition according to claim 4, wherein the intestinal bacteria are any one or more of live intestinal bacteria; genetically recombined, altered or modified, attenuated, chemically treated, physically treated, or inactivated intestinal bacteria; lysate of intestinal bacteria; and/or culture supernatant of intestinal bacteria.

6. Use of any one or more of intestinal bacteria selected from a group consisting of *Alistipes shahii, Eubacterium ramulus, Eubacterium siraeum, Prevotella ruminicola, Ruminococcu lactaris*, *Anaerofustis stercorihominis, Clostridium nexile*, *Sanguibacteroides justesenii, Dorea formicigenerans*, and *Bifidobacterium saeculare* in preparation of a medicament for promoting an accumulation of CD8⁺ cytotoxic T lymphocytes.

7. The use according to claim 6, wherein the intestinal bacteria are any one or more of live intestinal bacteria; genetically recombined, altered or modified, attenuated, chemically treated, physically treated, or inactivated intestinal bacteria; lysate of intestinal bacteria; and/or culture supernatant of intestinal bacteria.

8. The use according to claim 1, wherein the accumulation of CD8 positive cytotoxic T lymphocytes is an increase in a relative amount of CD8 positive cytotoxic T lymphocytes in tumor microenvironment.

9. A composition for promoting an accumulation of CD8 positive cytotoxic T lymphocytes, comprising any one or more of intestinal bacteria selected from a group consisting of *Alistipes shahii, Eubacterium ramulus, Eubacterium siraeum, Prevotella ruminicola, Ruminococcu lactaris*, *Anaerofustis stercorihominis, Clostridium nexile*, *Sanguibacteroides justesenii, Dorea formicigenerans*, and *Bifidobacterium saeculare* as an active ingredient.

10. The composition according to claim 9, wherein the intestinal bacteria are any one or more of live intestinal bacteria; genetically recombined, altered or modified, attenuated, chemically treated, physically treated, or inactivated intestinal bacteria; lysate of intestinal bacteria; and/or culture supernatant of intestinal bacteria.

11. Use of any one or more of intestinal bacteria selected from a group consisting of *Alistipes shahii, Eubacterium ramulus, Eubacterium siraeum, Prevotella ruminicola, Ruminococcu lactaris*, *Anaerofustis stercorihominis, Clostridium nexile*, *Sanguibacteroides justesenii, Dorea formicigenerans*, and *Bifidobacterium saeculare* in preparation of a medicament for preventing and/or treating tumors.

12. The use according to claim 11, wherein the intestinal bacteria are any one or more of live intestinal bacteria; genetically recombined, altered or modified, attenuated, chemically treated, physically treated, or inactivated intestinal bacteria; lysate of intestinal bacteria; and/or culture supernatant of intestinal bacteria.

13. The use according to claim 11, wherein the tumor is a solid tumor.

14. A composition for preventing and/or treating tumors, comprising any one or more of intestinal bacteria selected from a group consisting of *Alistipes shahii, Eubacterium ramulus, Eubacterium siraeum, Prevotella ruminicola, Ruminococcu lactaris*, *Anaerofustis stercorihominis, Clostridium nexile*, *Sanguibacteroides justesenii, Dorea formicigenerans*, and *Bifidobacterium saeculare* as an active ingredient.

15. The composition according to claim 14, wherein the intestinal bacteria are any one or more of live intestinal bacteria; genetically recombined, altered or modified, attenuated, chemically treated, physically treated, or inactivated intestinal bacteria; lysate of intestinal bacteria; and/or culture supernatant of intestinal bacteria.
